# EUROPEAN PATENT APPLICATION

(11) **EP 4 273 123 A1**
(43) Date of publication of application: **08.11.2023**
(21) Application number: 21915888.8
(22) Date of filing: 30.12.2021
(51) Int. Cl.: C07C 263/10, C07C 263/16, C07C 263/20, C07C 265/14

(54) **METHOD FOR PREPARING ISOCYANATE COMPOUND**

(30) Priority: 30.12.2020 KR 20200187829
(71) Applicant: Hanwha Solutions Corporation, Jung-gu Seoul 04541 (KR)
(72) Inventor: RYU, Hyun Cheol, Daejeon 34128 (KR); MIN, Ju Won, Daejeon 34128 (KR); HAN, Keedo, Daejeon 34128 (KR)
(74) Representative: Berggren Oy
(86) International application number: PCT/KR2021/020331
(87) International publication number: WO 2022/146091

(57) **Abstract**

The present invention relates to a method for preparing an isocyanate compound. According to the present invention, provided is a method for preparing an isocyanate compound that can minimize thermal denaturation of a reaction product and formation of by-products and obtain a high-purity isocyanate compound more stably during the preparation of an isocyanate compound using phosgene.

## Description

### [TECHNICAL FIELD]

The present invention relates to a method for preparing an isocyanate compound.

### [BACKGROUND ART]

Although xylylene diisocyanate (hereinafter, referred to as XDI) contains an aromatic ring in its molecule, it is classified into aliphatic isocyanate. XDI is a compound very useful for raw materials such as polyurethane-based materials, polyurea-based materials, or polyisocyanurate-based materials in the fields of chemical industry, resin industry, and paint industry.

Commonly, when synthesizing isocyanate compound, many side reactions are generated, and thus, it is prepared by a method of reacting with anhydrous hydrochloric acid or carbonic acid to form a salt of amine compound and reacting it with phosgene.

As an example, XDI is prepared by reacting xylylene diamine (hereinafter referred to as XDA) with anhydrous hydrochloric acid to form an amine-hydrochloride salt, and then reacting it with phosgene. More specifically, in the prior art, an isocyanate compound such as XDI was prepared by reacting a liquid raw amine, such as XDA-containing solution, with anhydrous hydrochloric acid to form XDA-HCl hydrochloride, heating it to a high temperature of at least 100°C or more, and then charging gas phase phosgene to progress a gas-liquid reaction.

The reaction of forming the isocyanate compound is a representative endothermic reaction, continuous heating and maintenance of a high temperature are required during the reaction so as to increase the yield.

By the way, since isocyanate compound such as XDI generally has high reactivity of the amino group, many side reactions are generated during the phosgenation reaction, and the by-products formed through the side reactions have an influence on a process in which isocyanate compounds are applied as raw materials (for example, a process for producing a polyurethane resin), thereby causing deterioration in resin quality.

As explained above, due the need to maintain a high temperature during the preparation process of isocyanate compound, and the high reactivity of the prepared isocyanate compound, such as XDI, there is a growing concern about the formation of by-products or the occurrence of side reactions by the thermal denaturation of products, and thus, high load has been frequently generated even in the purification process.

Due to such problems, various attempts have been made in the past to suppress side reactions or by-product generations during the preparation of isocyanate compound, but effective technology has not yet been developed.

### [DETAILED DESCRIPTION OF THE INVENTION]

### [Technical Problem]

It is an object of the present invention to provide a method for preparing an isocyanate compound that can minimize thermal denaturation of a reaction product and formation of by-products and obtain a high-purity isocyanate compound more stably during the preparation of an isocyanate compound using phosgene.

### [Technical Solution]

According to one embodiment of the present invention, there is provided a method for preparing isocyanate compound, comprising:
a reaction step of reacting a salt of amine compound with phosgene in the presence of a solvent to obtain a reaction product containing an isocyanate compound,
a degassing step of removing a gas phase from the reaction product,
a desolvation step of removing a solvent from the reaction product from which the gas phase has been removed,
a low boiling material-removing step of removing low boiling materials (i.e., lights) from the reaction product from which the solvent has been removed, and
a high boiling material-removing step of removing high boiling materials (i.e., heavies) from the reaction product from which the low boiling materials have been removed;
wherein the low boiling material-removing step is progressed in a distillation column to which a reboiler is connected to the bottom of the column, and overheated inert gas is supplied to the bottom of the distillation column through the reboiler.

Now, a method for preparing an isocyanate compound according to one embodiment of the present invention will be described.

Unless defined otherwise, all technical and scientific terms used herein have the same meaning as commonly understood by one of ordinary skill in the art to which this invention belongs. The terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

While the present invention can be modified in various ways and take on various alternative forms, specific embodiments thereof are illustrated and described in detail below. However, it should be understood that there is no intent to limit the present invention to the particular forms disclosed, but on the contrary, the present invention covers all modifications, equivalents, and alternatives falling within the spirit and scope of the present invention.

In describing a position relationship, for example, when the position relationship is described as 'upon~', 'above~', 'below~', and 'next to~', one or more portions may be arranged between two other portions unless `just' or 'direct' is used.

In describing a time relationship, for example, when the temporal order is described as 'after~', 'subsequent~', 'next~', and 'before~', a case which is not continuous may be included unless `just' or 'direct' is used.

As used herein, the term "low boiling material" means a material having a boiling point lower than that of the isocyanate compound which is a target product according to the present invention, and the term "high boiling material" means a material having a boiling point higher than that of the isocyanate compound, which is a target product according to the present invention.

As used herein, the term "bottom" of the distillation column means an outlet through which the lower discharged products of the distillation column are discharged to the outside of the distillation column, the term "top" of the distillation column means an outlet through which the upper discharged products of the distillation column are discharged to the outside of the distillation column.

According to one embodiment of the present invention, there is provided a method for preparing isocyanate compound, comprising:
a reaction step of reacting a salt of amine compound with phosgene in the presence of a solvent to obtain a reaction product containing an isocyanate compound,
a degassing step of removing a gas phase from the reaction product,
a desolvation step of removing a solvent from the reaction product from which the gas phase has been removed,
a low boiling material-removing step of removing low boiling materials (i.e., lights) from the reaction product from which the solvent has been removed, and
a high boiling material-removing step of removing high boiling materials (i.e., heavies) from the reaction product from which the low boiling materials have been removed;
wherein the low boiling material-removing step is progressed in a distillation column to which a reboiler is connected to the bottom of the column, and overheated inert gas is supplied to the bottom of the distillation column through the reboiler.

High purity is required to use isocyanate compounds as raw materials for fine chemical products including optical materials.

However, generally, when synthesizing an isocyanate compound including XDI, there is a problem that many side reactions occur, and the purity of the isocyanate compound is lowered due to reaction between reaction products and thermal denaturation.

In one example, aliphatic diisocyanate can be prepared by reacting an aliphatic diamine with phosgene. At this time, a side reaction occurs, and as a side reaction product, for example, a monoisocyanate such as (chloromethyl)benzyl isocyanate is produced. It is known that the occurrence of such side reactions and the formation of by-products are caused by the need to maintain a high temperature during the preparation process of aliphatic diisocyanate and the high reactivity of the prepared aliphatic diisocyanate such as XDI. In particular, the final product, aliphatic diisocyanate, causes side reactions when exposed to high temperatures for a certain period of time, or can form high-molecular-weight by-products, such as oligomers or polymers, including dimers or trimers or higher oligomers.

When the distillation column is operated at a low temperature so that the thermal decomposition does not occur, the pressure of the reboiler must be very low in order to boil the reaction product and form a reflux. However, considering the hydraulic parameters of the distillation column, it is very difficult to stably operate while maintaining a low pressure of 10 torr or less.

The present inventors have found, as a result of continuous research, that the reaction step, the degassing step, the desolvation step, the low boiling material-removing step, and the high boiling material-removing step are progressed in this order to prepare an isocyanate compound, especially when inert gas is supplied to the lower part of the distillation column through a reboiler connected to the distillation column in the low boiling material-removing step, the difference in hydraulic parameters between the column top and the column bottom of the distillation column can be reduced, so that the purification process can be operated more stably.

When the above steps are progressed under the above temperature and pressure conditions, thermal denaturation of a reaction product and formation of by-products can be minimized, and a high-purity isocyanate compound can be obtained.

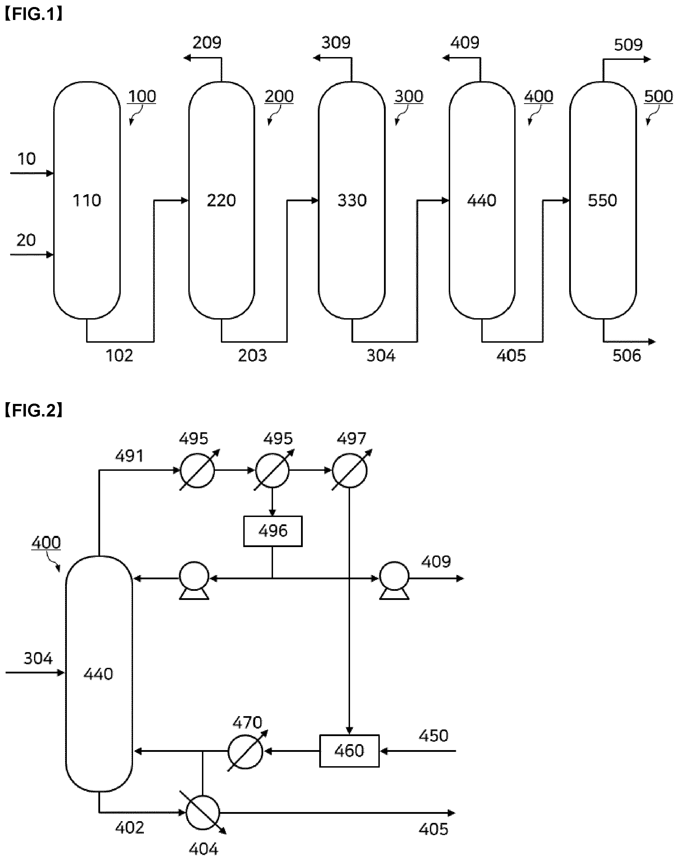

FIG. 1 schematically shows the process and apparatus utilized in the method for preparing an isocyanate compound according to an embodiment of the present invention.

Each step that can be included in the method for preparing an isocyanate compound according to an embodiment of the invention will be described below with reference to FIG. 1.

### (Reaction Step)

First, a reaction step is progressed in which a salt of amine compound and phosgene are reacted in the presence of a solvent to obtain a reaction product containing an isocyanate compound, the solvent, and unreacted phosgene.

According to one embodiment of the invention, the amine compound is preferably applied as a salt of the amine compound in order to suppress rapid reactions and side reactions between the amine compound and phosgene. For example, the salt of amine compound may be a hydrochloride or carbonic acid salt of the amine compound.

The salt of the amine compound may be prepared by reacting an amine compound with anhydrous hydrochloric acid or carbonic acid and performing a neutralization reaction. The neutralization reaction can be progressed at a temperature of 20 to 80°C.

The supply ratio of the anhydrous hydrochloric acid or carbonic acid may be, for example, 2 to 10 moles, 2 to 6 moles, or 2 to 4 moles, based on 1 mole of the amine compound.

Preferably, the amine compound may be a fatty amine having an aliphatic group in its molecule. Specifically, the aliphatic amine may be a chained or cyclic aliphatic amine. More specifically, the aliphatic amine may be a bifunctional or higher chained or cyclic aliphatic amine containing at least two amino groups in its molecule.

For example, the amine compound may be at least one compound selected from the group consisting of hexamethylenediamine, 2,2-dimethylpentanediamine, 2,2,4-trimethylhexanediamine, butenediamine, 1,3-butadiene-1,4-diamine, 2,4,4-trimethylhexamethylenediamine, 1,6,11-undecatriamine, 1,3,6-hexamethylenetriamine, 1,8-diisocyanato-4-(isocyanatomethyl)octane, bis(aminoethyl)carbonate, bis(aminoethyl)ether, xylylenediamine, α,α,α',α'-tetramethylxylylenediamine, bis(aminoethyl)phthalate, bis(aminomethyl)cyclohexane, dicyclohexylmethanediamine, cyclohexanediamine, methylcyclohexanediamine, dicyclohexyldimethylmethanediamine, 2,2-dimethyldicyclohexylmethanediamine, 2,5-bis(aminomethyl)bicyclo-[2,2,1]-heptane, 2,6-bis(aminomethyl)bicyclo-[2,2,1]-heptane, 3,8-bis(aminomethyl)tricyclodecane, 3,9-bis(aminomethyl)tricyclodecane, 4,8-bis(aminomethyl)tricyclodecane, 4,9-bis(aminomethyl)tricyclodecane, bis(aminomethyl)norbornene, and xylylenediamine.

Further, the amine compound may be at least one sulfur-containing aliphatic amine selected from the group consisting of bis(aminomethyl)sulfide, bis(aminoethyl)sulfide, bis(aminopropyl)sulfide, bis(aminohexyl)sulfide, bis(aminomethyl)sulfone, bis(aminomethyl)disulfide, bis(aminoethyl)disulfide, bis(am inopropyl)disulfide, bis(aminomethylthio)methane, bis(aminoethylthio)methane, bis(aminoethylthio)ethane, bis(aminomethylthio)ethane, and 1,5-diamino-2-aminomethyl-3-thiapentane.

Among the above amine compounds, xylylenediamine (XDA) can exhibit superior effects when applied to the method for preparing an isocyanate compound according to one embodiment of the invention. Preferably, the amine compound may be at least one compound selected from the group consisting of m-xylylenediamine, p-xylylenediamine and o-xylylenediamine.

According to an embodiment of the invention, the reaction step is progressed in a gas-liquid-solid three-phase reaction in which a salt of solid-phase amine compound and gas phase phosgene are reacted in the presence of a solvent. Accordingly, rapid reactions can be effectively suppressed, and side reactions and formation of by-products can be minimized.

When the salt of amine compound and phosgene are reacted in the presence of a solvent, phosgene may be charged at a time, or may be charged dividedly. For example, a small amount of phosgene can be primarily charged at a relatively low temperature, and reacted with the salt of amine compound to produce the intermediate. Subsequently, the remaining amount of phosgene can be secondarily charged at a relatively high temperature, and reacted with the intermediate to obtain a reaction solution containing an isocyanate compound. As an example, xylylene diamine and a small amount of phosgene are reacted to produce an intermediate in the form of a carbamoyl salt, to which the remaining amount of phosgene is then charged, and the intermediate in the form of a carbamoyl salt can be reacted with phosgene to form an aliphatic isocyanate such as xylylene diisocyanate.

Through such reaction steps, a time during which the final product isocyanate compound is exposed to high temperature can be minimized. Furthermore, the intermediate is formed at a relatively low temperature, thereby reducing a time during which a high temperature should be maintained in the whole reaction process. In addition, the amount of heat charged to the whole process can be reduced. Further, the high-temperature reaction time of phosgene can be relatively shortened, and the risk of explosive vaporization of phosgene can also be reduced.

The reaction step is progressed in the presence of a solvent containing at least one of an aromatic hydrocarbon-based solvent and an ester-based solvent. The solvent may be selected in consideration of the temperature at which the reaction step is progressed.

The aromatic hydrocarbon-based solvent may be a halogenated aromatic hydrocarbon-based solvent such as monochlorobenzene, 1,2-dichlorobenzene and 1,2,4-trichlorobenzene.

The ester-based solvent may be fatty acid esters such as amyl formate, n-butyl acetate, isobutyl acetate, n-amyl acetate, isoamyl acetate, methylisoamyl acetate, methoxybutyl acetate, sec-hexyl acetate, 2-ethylbutyl acetate, 2-ethylhexyl acetate, cyclohexyl acetate, methylcyclohexyl acetate, benzyl acetate, ethyl propionate, n-butyl propionate, isoamyl propionate, ethyl acetate, butyl stearate, butyl lactate, and amyl lactate; or aromatic carboxylic acid esters such as methyl salicylate, dimethyl phthalate and methyl benzoate.

According to one embodiment of the invention, the salt of amine compound in the reaction step may be contained in the solvent at a concentration of 20% by volume or less, for example, 1 to 20% by volume, or 5 to 20% by volume. When the concentration of the salt of amine compound contained in the solvent exceeds 20% by volume, a large amount of salt is likely to precipitate in the reaction step.

The reaction step may be progressed at a temperature of 165°C or less, preferably 80°C to 165°C.

According to one embodiment of the invention, when phosgene is dividedly charged in the reaction step, it can be charged in an amount of 10 to 30% by weight, or 12 to 30% by weight, or 15 to 28% by weight based on the entire amount of phosgene charged at a temperature of 80°C to 100°C or 85°C to 95°C. Under such reaction conditions, rapid reactions are suppressed and an intermediate in the form of a carbamoyl-based salt can be selectively and effectively formed. Subsequently, while charging the remaining amount of phosgene at a temperature of 110°C to 165°C or 120°C to 150°C, the intermediate and the phosgene can be reacted to obtain a reaction product containing an isocyanate compound.

The isocyanate compound may vary depending on the type of the amine compound used in the reaction step. For example, the isocyanate compound may be at least one compound selected from the group consisting of n-pentyl isocyanate, 6-methyl-2-heptane isocyanate, cyclopentyl isocyanate, hexamethylene diisocyanate(HDI), isophorone diisocyanate(IPDI), diisocyanatomethylcyclohexane(H6TDI), xylylene diisocyanate(XDI), diisocyanatocyclohexane(t-CHDI) and di(isocyanatocyclohexyl)methane(H12MDI).

In particular, the method for preparing an isocyanate compound according to an embodiment of the invention may be further useful for the preparation of xylylene diisocyanate (XDI). XDI includes 1,2-XDI (o-XDI), 1,3-XDI (m-XDI), and 1,4-XDI (p-XDI) as structural isomers.

The reaction step can be progressed either batchwise or continuously. The reaction step can be progressed in a reaction unit 100 that includes a reactor 110 having a rotating shaft; an amine compound salt supply line 10 and a phosgene supply line 20 that supply reactants to the inside of the reactor; a heat source that supplies a heat quantity to the reactor; and a reaction product transfer line 102 that transfers the reaction product obtained in the reactor to a subsequent purification step.

### (Degassing step)

Then, a degassing step of removing the gas phase from the reaction product is progressed.

In the degassing step, a gas phase such as surplus phosgene or byproduct hydrogen chloride is removed from the reaction product by a known degassing unit 200.

A method of removing the gas phase from the reaction product may include a method of supplying and aerating gas, or a method of separating the gas phase from the reaction product using a flash tank or a distillation column.

In particular, according to an embodiment of the invention, in order to minimize the formation of by-products in the degassing step, the degassing step is preferably progressed at a temperature of 145°C to 165°C and a pressure of 100 torr to 600 torr.

In one example, the degassing step may be progressed in a distillation column, wherein setting the temperature at the bottom of the distillation column to 145°C to 165°C and operating the distillation column under a column top pressure of 100 torr to 600 torr may be advantageous to minimize the formation of by-products. Here, the temperature at the bottom of the distillation column may mean the temperature of a reboiler connected to the bottom of the distillation column. And, the pressure at the top of the distillation column may mean the pressure of a condenser connected to the top of the distillation column.

Preferably, the temperature at which the degassing step is progressed may be 145°C to 165°C, or 150°C to 165°C, or 150°C to 160°C, or 155°C to 160°C. And, preferably, the pressure at which the degassing step is progressed may be 100 torr to 600 torr, or 200 torr to 500 torr, or 300 torr to 450 torr, or 350 torr to 450 torr.

When the temperature and pressure at which the degassing step is progressed do not satisfy the above ranges, the formation of by-products in the degassing step can increase, and the removal efficiency of a gas phase can decrease, whereby the concentration of the isocyanate compound in the reaction product obtained from the degassing step can decrease.

As an example, the degassing step can be progressed in a degassing unit 200 including a distillation column 220 configured so as to be able to remove a gas phase from the reaction product supplied via the reaction product transfer line 102 of the reaction unit 100; a gas phase discharge line 209 that discharges a gas phase to the top of the distillation column; and a reaction product transfer line 203 that discharges the reaction product from which the gas phase has been removed to the bottom of the distillation column and transfers it to a subsequent step.

### (Desolvation step)

Then, a desolvation step may be progressed in which a solvent is removed from the reaction product from which the gas phase has been removed.

In the desolvation step, the solvent is distilled off from the reaction product from which the gas phase has been removed by a known desolvation unit 300.

According to one embodiment of the invention, in order to minimize thermal denaturation of the reaction product and formation of by-products in the desolvation step, the desolvation step is preferably progressed at a temperature of 100°C to 165°C and a pressure of 30 torr or less.

In one example, the desolvation step can be progressed in a distillation column, wherein setting the temperature at the bottom of the distillation column to 100°C to 165°C and operating the distillation column to a column top pressure of 30 torr or less can be advantageous to minimize the thermal degradation and the formation of by-products. Here, the temperature at the bottom of the distillation column may mean the temperature of a reboiler connected to the bottom of the distillation column. And, the pressure at the top of the distillation column may mean the pressure of a condenser connected to the top of the distillation column.

Preferably, the temperature at which the desolvation step is progressed may be 100°C to 165°C, or 115°C to 165°C, or 115°C to 160°C, or 130°C to 160°C. And, preferably, the pressure at which the desolvation step is progressed may be 5 torr to 30 torr, or 5 torr to 25 torr, or 10 torr to 20 torr, or 10 torr to 15 torr.

When the temperature and pressure at which the desolvation step is progreesed do not satisfy the above ranges, the thermal denaturation and the formation of by-products in the desolvation step may increase, and the solvent removal efficiency may decrease, whereby the concentration of the isocyanate compound in the reaction product obtained from the desolvation step may decrease.

In one example, the desolvation step can be progressed in a desolvation unit 300 that includes a distillation column 330 configured so as to be able to remove a solvent from the reaction product supplied via the reaction product transfer line 203 of the degassing unit 200; a solvent discharge line 309 that discharges a solvent to the top of the distillation column; and a reaction product transfer line 304 that discharges the reaction product from which the solvent has been removed to the bottom of the distillation column, and transfers it to a subsequent step.

In order to minimize the high temperature residence time of the reaction product in the desolvation step, a distillation column 440 equipped with apparatuses such as a kettle type reboiler, a thin-film evaporator, a force circulated reboiler, and a jacketed vessel type reboiler can be utilized.

### (Low boiling material-removing step)

Then, a low boiling material-removing step is progressed in which low boiling materials (i.e., lights) are removed from the reaction product from which the solvent has been removed.

In the low boiling material-removing step, the low boiling materials (lights) are removed by a known low boiling material-removing unit 400 from the reaction product from which the solvent has been removed.

The low boiling material means a material having a boiling point lower than that of the isocyanate compound, which is the main product in the reaction step. Examples of the low boiling materials include materials produced by side reactions in the process of obtaining the main product. As an example, in the process of preparing XDI as the isocyanate compound, the low boiling material may include monoisocyanate such as (chloromethyl)benzyl isocyanate (CBI) and ethylphenyl isocyanate (EBI).

According to one embodiment of the invention, in order to minimize the thermal denaturation of reaction products and the formation of by-products in the low boiling material-removing step, the low boiling material-removing step is preferably progressed under the conditions where the temperature at the bottom of the distillation column 440 is set to 160°C or less, and the pressure at the bottom of the distillation column is set to 50 torr or less. Here, the temperature at the bottom of the distillation column may mean the temperature of a reboiler connected to the bottom of the distillation column. And, the pressure at the bottom of the distillation column may mean the pressure of a reboiler connected to the bottom of the distillation column.

Preferably, the temperature at which the low boiling material-removing step is progressed may be 145°C to 165°C, or 150°C to 165°C, or 150°C to 160°C, or 155°C to 160°C. And, preferably, the pressure at which the low boiling material-removing step is progressed may be 1 torr to 50 torr, or 5 torr to 40 torr, or 10 torr to 30 torr.

If the temperature and pressure at which the low boiling material-removing step is progressed do not satisfy the above ranges, the thermal denaturation and the formation of by-products in the low boiling material-removing step may increase, and the removal efficiency of low boiling materials may decrease, whereby the concentration of isocyanate compounds in the reaction product obtained from the low boiling material-removing step can be reduced.

In one example, the low boiling material-removing step can be progressed in a low boiling material-removing unit 400 that includes a distillation column 440 configured so as to be able to remove low boiling materials from the reaction product supplied through the reaction product transfer line 304 of the desolvation unit 300; a low boiling material discharge line 409 that discharges low boiling materials to the top of the distillation column; and a reaction product transfer line 405 that discharges the reaction product from which the low boiling materials have been removed to the bottom of the distillation column, and transfers it to a subsequent step.

In particular, according to an embodiment of the invention, the low boiling material-removing step is progressed in a distillation column to which a reboiler is connected to the bottom of the column, and progressed such that overheated inert gas is supplied to the bottom of the column through the reboiler.

In order to minimize the thermal decomposition of the isocyanate compound contained in the reaction product in the low boiling material-removing step, the temperature of the lower part of the distillation column 440 should be maintained as low as 165°C or less. By the way, in order to boil the reaction product under the temperature condition to form a reflux, the pressure of the reboiler must be maintained as low as 10 torr or less. However, considering the hydraulic parameters of the distillation column 440, it is very difficult to stably operate the distillation column 440 while maintaining the pressure of the reboiler as low as 10 torr or less.

By the way, as in one embodiment of the invention, the low boiling material-removing step is progressed in a distillation tower to which a reboiler is connected to the bottom of the column, and when supplying overheated inert gas to the bottom of the column through the reboiler, the difference in hydraulic parameters between the column top and the column bottom of the distillation column can be reduced, so that the process can be operated more stably.

FIG. 2 schematically shows the process and apparatus used in the low boiling material-removing step in the method for preparing an isocyanate compound according to one embodiment of the invention.

Referring to FIG. 2, in the low boiling material-removing step, the reaction product obtained in the desolvation unit 300 is supplied to the distillation column 440 through a reaction product transfer line 304.

The low boiling material-enriched fraction in the distillation column 440 is transferred to the low boiling material condenser 495 through a column top stream line 491 connected to the top of the distillation column 440. The low boiling materials condensed in the condenser 495 are collected in a reflux drum 496, a part of the condensed low boiling materials are refluxed to the top of the distillation column 440, and the remaining low boiling materials are discharged through a low boiling material discharge line 409. The inert gas not condensed in the condenser 495 is condensed in an inert gas condenser 497, and transferred to an inert gas recovery drum 460.

The isocyanate compound-enriched fraction in the distillation column 440 is transferred to a reboiler 404 through the bottom stream line 402 connected to the bottom of the distillation column 440. In the reboiler 404, the low boiling fraction is re-supplied to the bottom of the distillation column 440, and the fraction containing the isocyanate compound is transferred to a high boiling material-removing unit 500 through a reaction product transfer line 405.

In particular, according to an embodiment of the invention, a fresh inert gas is further supplied to an inert gas recovery drum 460 through an inert gas supply line 450. The inert gas collected in the inert gas recovery drum 460 is overheated in an inert gas preheater 470. The overheated inert gas is charged into the reboiler 404 and supplied to the bottom of the distillation column 440.

A device such as a ring sparger is preferably used to charge the overheated inert gas into the reboiler 404. In particular, the bottom of the distillation column 440 is preferably provided with an internal baffle to prevent the overheated inert gas from mixing into the bottom of the column.

According to one embodiment of the invention, the inert gas may be a hydrocarbon-based gas having 1 to 6 carbon atoms. Specifically, the inert gas may be one or more gases selected from the group consisting of methane gas, ethane gas, propane gas, butane gas, pentene gas, and hexane gas.

The inert gas is preferably supplied in a state of being heated (overheated) by the temperature of the low boiling fraction obtained by being heated in the reboiler 404. Preferably, the inert gas may be supplied to the bottom of the distillation column 440 in a state of being overheated to a temperature of 150°C or more (preferably 155°C to 160°C) by the inert gas preheater 470.

When the inert gas thus overheated is supplied to the bottom of the distillation column 440, a part of the isocyanate compound and the low boiling material included in the reaction product can be pulled up to a low boiling material condenser 495 connected to the top of the distillation column 440. This flow can reduce the difference in hydraulic parameters between the top and bottom of the distillation column 440, thereby enabling stable process operation even under the temperature at the bottom of the distillation column.

In order to minimize the high temperature residence time of the reaction product in the low boiling material-removing step, the low boiling material-removing step is preferably progressed in a distillation column 440 to which a jacketed vessel type reboiler is connected to the bottom of the column.

### (High boiling material-removing step)

Then, a high boiling material-removing step is progressed in which high boiling materials (i.e., heavies) are removed from the reaction product from which the low boiling materials have been removed.

In the high boiling material-removing step, high boiling materials (i.e., heavies) are removed by a known high boiling material-removing unit 500 from the reaction product from which the low boiling materials have been removed.

The high boiling material means a material having a boiling point higher than that of the isocyanate compound, which is the main product in the reaction step. Examples of the high boiling materials include high-molecular-weight by-products such as oligomers or polymers including dimers, trimers or higher multimers of isocyanate compounds formed in the step of obtaining the main product.

According to one embodiment of the invention, in order to minimize the thermal denaturation of the reaction product and the formation of by-products in the high boiling material-removing step, the high boiling material-removing step may preferably be progressed at a temperature of 145°C to 165 °C and a pressure of 1 torr or less.

In one example, the high boiling material-removing step may be progressed under a thin-film evaporator, wherein setting the temperature at the bottom of the distillation column to 145°C to 165°C and operating the distillation column under a column top pressure of 1 torr or less can be advantageous to minimize the thermal degradation and the formation of by-products.

Preferably, the temperature at which the high boiling material-removing step is progressed may be 145°C to 165°C, or 150°C to 165°C, or 150°C to 160°C, or 155°C to 160°C. And, preferably, the pressure at which the high boiling material-removing step is progressed may be 0.1 torr to 1 torr, or 0.5 torr to 1 torr.

If the temperature and pressure at which the high boiling material-removing step is progressed do not satisfy the above ranges, the thermal denaturation and the formation of by-products in the high boiling material-removing step may increase, and the removal efficiency of high boiling materials may decrease, whereby the concentration of the isocyanate compound obtained from the high boiling material-removing step may decrease.

In one example, the high boiling material-removing step may be progressed in a high boiling material-removing unit 500 that includes a thin-film evaporator 550 configured so as to be able to remove high boiling materials from the reaction product supplied through the reaction product transfer line 405 of the low boiling material-removing unit 400; an isocyanate compound discharge line 509 that discharges an isocyanate compound to a condensing section of the thin-film evaporator; and a high boiling material discharge line 506 that discharges the high boiling material to the bottom of the thin-film evaporator.

### [Advantageous Effects]

According to the present invention, provided is a method for preparing an isocyanate compound that can minimize thermal denaturation of a reaction product and formation of by-products and obtain a high-purity isocyanate compound more stably during the preparation of an isocyanate compound using phosgene.

### [BRIEF DESCRIPTION OF THE DRAWINGS]

FIG. 1 schematically shows the process and apparatus used in the method for preparing an isocyanate compound according to one embodiment of the present invention.
FIG. 2 schematically shows the process and apparatus used in the low boiling material-removing step in the method for preparing an isocyanate compound according to one embodiment of the present invention.

### <Description of Reference Numerals>

10: amine compound salt supply line
20: phosgene supply line
100: reaction unit
110: reactor
102: reaction product transfer line
200: degassing unit
220 distillation column
209: gas phase discharge line
203: reaction product transfer line
300: desolvation unit
330: distillation column
309: solvent discharge line
304: reaction product transfer line
400: low boiling material-removing unit
440: distillation column
409: low boiling material discharge line
405: reaction product transfer line
402: bottom stream line
404: reboiler
450: inert gas supply line
460: inert gas recovery drum
470: inert gas preheater
491: column top stream line
495: low boiling material condenser
496: reflux drum
497: inert gas condenser
500: high boiling material-removing unit
550: thin-film evaporator
506: high boiling material discharge line
509: isocyanate compound discharge line

### [DETAILED DESCRIPTION OF THE EMBODIMENTS]

Hereinafter, the actions and effects of the invention will be explained in more detail through specific examples of the invention. However, these examples are presented only as the illustrations of the invention, and the scope of the right of the invention is not determined thereby.

### Example 1

1,3-XDI (m-XDI) was prepared by the following process using the apparatus shown in FIGS. 1 and 2.

### (Reaction step)

Under normal temperature and pressure conditions, 500 kg of hydrochloric acid and 560 kg of m-xylylenediamine (m-XDA) were reacted in 6500 kg of 1,2-dichlorobenzene (o-DCB) solvent for 4 hours to form 870 kg of the hydrochloride salt of m-xylylenediamine.

870 kg of the hydrochloride salt of m-xylylenediamine was charged into a reactor 110 through a supply line 10, and the temperature of the reactor was raised to 125°C. During the progress of the reaction, a total of 1359 kg of phosgene was put into the reactor through the supply line 20, and stirred. A dry ice-acetone condenser was used from the time of charging phosgene to the time of completion of the reaction to prevent phosgene from leaking to the outside. The reaction was allowed to progress for 1.5 hours at a temperature of 90°C.

After that, the reactor was heated so that the internal temperature thereof was 125°C, and 6000 kg of phosgene was further charged. The temperature of the reactor was maintained at 125°C, and further stirred for 4.5 hours until the reaction solution became clear. Heating was stopped after the reaction solution became clear. The reaction product obtained by the above method was transferred to the distillation unit 200 through a transfer line 102.

### (Degassing step)

The distillation column 220 of the degassing unit 200 was configured so as to be able to remove the gas phase from the reaction product supplied through the transfer line 102.

Specifically, the distillation column 220 used was a packing tower packed with Mellapak, which is a type of structural packing, and had a height of about 6 m.

The temperature at the bottom of the distillation column 220 was set to 155°C and the distillation column 220 was operated under a column top pressure of 400 torr.

About 150 kg/hr of the reflux stream was re-charged to a distillation column 220, which corresponds to a reflux ratio of about 3.2 relative to the column top outflow amount.

The gas phase containing phosgene was discharged through a gas phase discharge line 209 connected to the top of the distillation column 220. The reaction product from which the gas phase has been removed was transferred to the desolvation unit 300 through the transfer line 203 connected to the bottom of the distillation column 220.

### (Desolvation step)

The distillation column 330 of the desolvation unit 300 was configured so as to be able to remove the solvent from the reaction product supplied through the transfer line 203.

Specifically, the distillation column 330 used was a packing tower packed with Mellapak, which is a type of structural packing, and had a height of about 7 m.

The temperature at the bottom of the distillation column 330 was set to 160°C, and the distillation column 330 was operated under a column top pressure of 15 torr.

About 600 kg/hr of the reflux stream was re-charged to the distillation column 330, which corresponds to a reflux ratio of about 0.5 relative to the column top outflow amount.

A solvent including 1,2-dichlorobenzene (o-DCB) was discharged through a solvent discharge line 309 connected to the top of the distillation column 330. The reaction product from which the solvent has been removed was transferred to the degassing unit 400 through the transfer line 304 connected to the bottom of the distillation column 330.

### (Low boiling material-removing step)

The distillation column 440 of the high boiling material-removing unit 400 was configured so as to be able to remove low boiling materials from the reaction product supplied through the transfer line 304.

Specifically, the distillation column 440 used was a packing tower packed with Mellapak, which is a type of structural packing, and had a height of about 13 m.

The temperature at the bottom of the distillation column 440 was set to 160°C and the distillation column 440 was operated under a column bottom pressure of 30 torr.

About 120 kg/hr of the reflux stream was re-charged to the distillation column, which corresponds to a reflux ratio of about 0.8 relative to the column top outflow amount.

The low boiling material-enriched fraction in the distillation column 440 was transferred to a low boiling material condenser 495 through a column top stream line 491 connected to the top of the distillation column 440. The low boiling materials condensed in the condenser 495 are collected in a reflux drum 496, a part of the condensed low boiling materials were refluxed to the top of the distillation column 440, and the remaining low boiling materials were discharged through the low boiling material discharge line 409. The inert gas not condensed in the condenser 495 is condensed in an inert gas condenser 497 and transferred to an inert gas recovery drum 460.

The 1,3-XDI-enriched fraction in the distillation column 440 was transferred to the reboiler 404 through a bottom stream line 402 connected to the bottom of the distillation column 440. The low boiling fraction in the reboiler 404 was re-supplied to the bottom of the distillation column 440, and the fraction containing 1,3-XDI was transferred to the high boiling material-removing unit 500 through the reaction product transfer line 405.

A fresh inert gas (hexane) was further supplied to an inert gas recovery drum 460 through an inert gas supply line 450. The inert gas collected in the inert gas recovery drum 460 is overheated in an inert gas preheater 470. The inert gas overheated to 155°C was supplied to the bottom of the distillation column 440 together with the low boiling fraction obtained in the reboiler 404.

### (High boiling material-removing step)

The thin-film evaporator 550 of the high boiling material-removing unit 500 was configured so as to be able to remove high boiling materials from the reaction product supplied through the transfer line 405.

Specifically, the thin-film evaporator 550 used was an evaporator in the form of a short path evaporator having a condenser installed therein.

The bottom temperature of the thin-film evaporator 550 was set to 135°C, and the thin-film evaporator 550 was operated under an evaporator bottom pressure of 0.5 torr.

About 13 kg/hr of high boiling condensate was separated from the bottom of the thin-film evaporator.

Isocyanate compounds including 1,3-XDI (*m*-XDI) were discharged through the discharge line 509 of the thin-film evaporator 550. The high boiling materials separated from the reaction product were discharged through a discharge line 506 connected to the bottom of the thin-film evaporator 550.

### Example 2

An isocyanate compound including 1,3-XDI (*m*-XDI) was obtained in the same manner as in Example 1, except that in the low boiling material-removing step, the distillation column 440 was operated so that the temperature at the bottom thereof was 130°C.

### Example 3

An isocyanate compound including 1,3-XDI (*m*-XDI) was obtained in the same manner as in Example 1, except that in the low boiling material-removing step, the distillation column 440 was operated so that the pressure at the bottom thereof was 20 torr.

### Comparative Example 1

An isocyanate compound including 1,3-XDI (*m*-XDI) was obtained in the same manner as in Example 1, except that in the low boiling material-removing step, the distillation column 440 was operated at a bottom temperature of 160°C and a bottom pressure of 30 torr without charging an inert gas (hexane) into the bottom of the column.

### Test Example

Part of the reaction product obtained in the low boiling material-removing step of Examples and Comparative Example was recovered, and subjected to gel permeation chromatography analysis. The contents of the main components identified through the above analysis are shown in Table 1 below.

In addition, the energy per unit time (kcal/hr) charged to the scrubbing step and the distillation step of Examples and Comparative Examples was measured, and shown in Tables 1 to 5 below.

**[Table 1]**

| (wt%) | oDCB | Lights | m-XDI | Hexane |
|---|---|---|---|---|
| Example 1 | - | 0.02 | 89.6 | - |
| Example 2 | - | 0.4 | 88.7 | - |
| Example 3 | - | 0.01 | 89.7 | - |
| Comparative Example 1 | 7.2 | 1.9 | 80.5 | - |

| | | | | |
|---|---|---|---|---|
| *oDCB: 1,2-dichlorobenzene | | | | |

Referring to Table 1, it was confirmed that the method for preparing an isocyanate compound according to Examples can minimize thermal denaturation of the reaction product and formation of by-products, and can obtain a high-purity isocyanate compound more stably.

On the contrary, it was confirmed that in the case of Comparative Example 1, in the low boiling material-removing step, the column was operated under a column bottom pressure of 30 torr as in Examples, but the concentration of the obtained isocyanate compound was remarkably low.

## Claims

1. A method for preparing an isocyanate compound, comprising:
a reaction step of reacting a salt of amine compound with phosgene in the presence of a solvent to obtain a reaction product containing an isocyanate compound,
a degassing step of removing a gas phase from the reaction product,
a desolvation step of removing a solvent from the reaction product from which the gas phase has been removed,
a low boiling material-removing step of removing low boiling materials from the reaction product from which the solvent has been removed, and
a high boiling material-removing step of removing high boiling materials from the reaction product from which the low boiling materials have been removed;
wherein the low boiling material-removing step is progressed in a distillation column to which a reboiler is connected to the bottom of the column, and overheated inert gas is supplied to the bottom of the distillation column through the reboiler.

2. The method of claim 1, wherein the low boiling material-removing step is progressed by a process in which inert gas overheated in an inert gas preheater is charged into the reboiler using a ring sparger.

3. The method of claim 1, wherein the inert gas is a hydrocarbon-based gas having 1 to 6 carbon atoms.

4. The method of claim 1, wherein the inert gas is overheated to a temperature of 150°C or more.

5. The method of claim 1, wherein the above steps are each progressed under a temperature of 165°C or less.

6. The method of claim 1, wherein the low boiling material-removing step is progressed so that the temperature at the bottom of the distillation column is 165°C or less and the pressure at the bottom is 50 torr or less.

7. The method of claim 1, wherein the reboiler is a jacketed vessel type reboiler.

8. The method of claim 1, wherein the distillation column in the low boiling material-removing step is provided with an internal baffle to prevent the overheated inert gas from mixing into the bottom of the distillation column.

9. The method of claim 1, wherein the amine compound is an aliphatic amine having an aliphatic group in its molecule.

10. The method of claim 1, wherein the amine compound is a bifunctional or higher chained or cyclic aliphatic amine containing two or more amino groups in its molecule.

11. The method of claim 1, wherein the amine compound is at least one compound selected from the group consisting of hexamethylenediamine, 2,2-dimethylpentanediamine, 2,2,4-trimethylhexanediamine, butenediamine, 1,3-butadiene-1,4-diamine, 2,4,4-trimethylhexamethylenediamine, 1,6,11-undecatriamine, 1,3,6-hexamethylenetriamine, 1,8-diisocyanato-4-(isocyanatomethyl)octane, bis(aminoethyl)carbonate, bis(aminoethyl)ether, xylylenediamine, α,α,α',α'-tetramethylxylylenediamine, bis(aminoethyl)phthalate, bis(aminomethyl)cyclohexane, dicyclohexylmethanediamine, cyclohexanediamine, methylcyclohexanediamine, dicyclohexyldimethylmethanediamine, 2,2-dimethyldicyclohexylmethanediamine, 2,5-bis(aminomethyl)bicyclo-[2,2,1]-heptane, 2,6-bis(aminomethyl)bicyclo-[2,2,1]-heptane, 3,8-bis(aminomethyl)tricyclodecane, 3,9-bis(aminomethyl)tricyclodecane, 4,8-bis(aminomethyl)tricyclodecane, 4,9-bis(aminomethyl)tricyclodecane, bis(aminomethyl)norbornene, and xylylenediamine.

12. The method of claim 1, wherein the amine compound is at least one sulfur-containing aliphatic amine selected from the group consisting of bis(aminomethyl)sulfide, bis(aminoethyl)sulfide, bis(aminopropyl)sulfide, bis(aminohexyl)sulfide, bis(aminomethyl)sulfone, bis(aminomethyl)disulfide, bis(aminoethyl)disulfide, bis(am inopropyl)disulfide, bis(aminomethylthio)methane, bis(aminoethylthio)methane, bis(aminoethylthio)ethane, bis(aminomethylthio)ethane, and 1,5-diamino-2-aminomethyl-3-thiapentane.

13. The method of claim 1, wherein the amine compound is at least one compound selected from the group consisting of m-xylylenediamine, p-xylylenediamine and o-xylylenediamine.

14. The method of claim 1, wherein the salt of amine compound is a hydrochloride or carbonic acid salt of the amine compound.

15. The method of claim 1, wherein the reaction step is progressed in the presence of a solvent containing at least one of an aromatic hydrocarbon-based solvent and an ester-based solvent.
